(19) Europäisches Patentamt
European Patent Office
Office européen des brevets

(11) Publication number: **0 572 057 A1**

## EUROPEAN PATENT APPLICATION

(21) Application number: 93201281.8

(22) Date of filing: 06.05.93

(51) Int. Cl.⁵: **C12Q 1/68**, C12Q 1/70

The applicant has subsequently filed a sequence listing and declared, that it includes no new matter.

(30) Priority: **11.05.92 US 880911**

(43) Date of publication of application:
**01.12.93 Bulletin 93/48**

(84) Designated Contracting States:
**AT BE CH DE DK FR GB IE IT LI LU NL SE**

(71) Applicant: **EASTMAN KODAK COMPANY**
**343 State Street**
**Rochester, New York 14650-2201(US)**

(72) Inventor: **Yoo, Youngtai, c/o Eastman Kodak Company**
**Patent Department,**
**343 State Street**
**Rochester, New York 14650-2201(US)**
Inventor: **Sharkey, David John, c/o Eastman Kodak Company**
**Patent Department,**
**343 State Street**
**Rochester, New York 14650-2201(US)**
Inventor: **Christy, Kenneth George, c/o Eastman Kodak Company**
**Patent Department,**
**343 State Street**
**Rochester, New York 14650-2201(US)**
Inventor: **Esders, Theodore Walter, c/o Eastman Kodak Company**
**Patent Department,**
**343 State Street**
**Rochester, New York 14650-2201(US)**

(74) Representative: **Nunney, Ronald Frederick Adolphe**
**Kodak Limited**
**Patent Department**
**Headstone Drive**
**Harrow Middlesex HA1 4TY (GB)**

(54) **PCR reagent composition, test kit and methods for amplification and detection with reduced nonspecific amplification of nucleic acids.**

(57) A PCR reagent can be encapsulated with an inert, meltable material which will keep the reagent from interaction in unwanted amplification reactions until its reaction is desired in the amplification of a targeted nucleic acid. Various reagents used in PCR can be so encapsulated, but particularly the DNA polymerase coreagents and deoxyribonucleotide-5'-triphosphates. The encapsulating material is a water-impermeable solid at room temperature, but melts at a temperature of at least about 50°C at which primer extension products are generally formed. The encapsulated PCR reagent can be used in various amplification and detection methods to reduce nonspecific amplifications.

This invention relates to diagnostic test kits and methods useful for amplification of nucleic acids using polymerase chain reaction (PCR) procedures. More specifically, the present invention relates to amplification and detection methods and test kits which can be used to greatly reduce nonspecific amplification of undesirable nucleic acids, or what is known in the art as "zero-cycle artifacts". This invention is particularly directed to the field of nucleic acid diagnostics.

Technology to detect minute quantities of nucleic acids has advanced rapidly over the last two decades including the development of highly sophisticated hybridization assays using probes in amplification techniques such as PCR. Researchers have readily recognized the value of such technology to detect diseases and genetic features in human or animal test specimens. The use of probes and primers in such technology is based upon the concept of complementarity, that is the bonding of two strands of a nucleic acid by hydrogen bonds between complementary nucleotides (also known as nucleotide pairs).

PCR is a significant advance in the art to allow detection of very small concentrations of a targeted nucleic acid. The details of PCR are described, for example, in US-A-4,683,195, US-A-4,683,202 and US-A-4,965,188, although there is a rapidly expanding volume of literature in this field. Without going into extensive detail, PCR involves hybridizing primers to the strands of a targeted nucleic acid (considered "templates") in the presence of a polymerization agent (such as a DNA polymerase) and deoxyribonucleoside triphosphates under the appropriate temperature, pH and concentrations. The result is the formation of primer extension products along the templates, the products having added thereto nucleotides which are complementary to the templates.

Once the primer extension products are denatured, one copy of the templates has been prepared, and the cycle of priming, extending and denaturation can be carried out as many times as desired to provide an exponential increase in the amount of nucleic acid which has the same sequence as the targeted nucleic acid. In effect, the targeted nucleic acid is duplicated (or "amplified") many times so that it is more easily detected. Despite the broad and rapid use of PCR in a variety of biological and diagnostic fields, there are still practical limitations which must be overcome to achieve the optimum success of the technology. PCR also exhibits considerable inefficiency in the use of expensive reagents.

Many amplification procedures yield nonspecific side products of nucleic acids that are not targeted. Sometimes nonspecificity is caused by mispriming by the primers so that they anneal to non-targeted nucleic acids. Many PCR procedures also yield primer dimers or oligomers and double-stranded side products containing the sequences of several primer molecules joined end-to-end. All of these unwanted products adversely affect accurate and sensitive detection of the targeted nucleic acid.

The problem caused by unwanted side products is particularly acute when the targeted nucleic acid is present in very low concentrations, for example, less than 1000 molecules. Such nucleic acids are generally considered "low-copy" nucleic acids, and can arise from early stages of infectious diseases or because of a very small specimen, such as may be the situation with forensic investigations.

The high sensitivity of PCR makes the process especially susceptible to contamination where amplified targeted nucleic acid from one reaction is transferred into subsequent reactions using the same primers, generating a false positive in the later reactions.

Under ideal conditions for PCR, the primers used will bind very specifically to the targeted nucleic acid only, particularly at elevated temperatures used in the process. However, the reaction mixture may also be held at lower temperatures at certain times (for example, during manufacture, shipping or before use by a customer), and the primers may undesirably bind to the non-targeted nucleic acids. If this occurs, nonspecific primer extension products and primer dimers can form which can be amplified along with the target nucleic acid during PCR cycles at elevated temperatures. These undesired products can obscure any amplified target nucleic acid (that is, produce high background). The primers are less efficient in amplification of the target nucleic acid, and thus the process requires more of the highly expensive reagents to produce an accurate result. Because reagents in the reaction are utilized to make non-specific products, less specific product is produced, rendering the process less sensitive for target nucleic acid.

WO 91/12342 describes amplification procedures in which the PCR reagents are mixed in certain vessels in such a manner that nonspecific product formation is apparently minimized. The reagents are in part separated from each other until amplification is desired using barriers of a wax or grease in a reaction or storage vessel. In some instances, the PCR reagents are embedded within the wax or grease, or in a gel or matrix. A magnesium compound essential in preferred PCR using thermostable DNA polymerases is preferably kept separate from the other PCR reagents in such a manner.

This teaching provides some advantages in certain contexts of PCR, but has a number of disadvantages as well, including the possibility of leakage through the wax or grease barrier. To reduce this possibility, a tedious aging test is proposed. Moreover, the magnesium salts used in the PCR are fatty acid salts which presumably are needed for compatibility with the barrier and to reduce leakage (that is, less diffusibility). The use of such a "macro-barrier" of wax or grease seems adaptable to certain specialized reaction vessels which may not be readily available to every PCR operator. Moreover, the reaction vessel must be such that a good seal can be maintained between it and the wax or grease barrier to minimize leakage. These particular reaction vessels may not be suitable for a given PCR cycling apparatus.

It would be highly desirable to eliminate the problem of non-targeted nucleic acids being amplified and formation of primer dimers without having to actually separate them from the targeted nucleic acid. It would also be desirable to avoid the problems attendant with the techniques described in WO 91/12342 (noted above).

The problems described above have been overcome with the use of a PCR reagent composition comprising a PCR reagent, the composition characterized wherein the PCR reagent is encapsulated within an inert material which is a water-impermeable solid at room temperature, but which melts at a temperature of at least 50°C.

This invention also provides a diagnostic test kit for polymerase chain reaction comprising, individually packaged, two or more PCR reagents, the kit characterized wherein at least one of the PCR reagents is encapsulated as described above.

This invention also provides a method for the amplification of a target nucleic acid comprising the steps of:

A. contacting a specimen suspected of containing a target nucleic acid with the following PCR reagents, in any order,

1) a primer complementary to the target nucleic acid,
2) a DNA polymerase,
3) a DNA polymerase cofactor, and
4) a deoxyribonucleotide-5'-triphosphate, provided at least one of the PCR reagents 1) to 4) is encapsulated within an inert material which is a water-impermeable solid at room temperature, but which melts at a temperature of at least 50°C, and

B. heating the resulting mixture of the specimen and the encapsulated PCR reagent at a temperature sufficient to melt the encapsulating material and to form the primer extension product, the temperature being at least 50°C.

Moreover, a method for the amplification and detection of a target double-stranded nucleic acid comprises the steps of:

A. heating a target double-stranded nucleic acid to denature its strands,
B. in the presence of the following PCR reagents:

1) a set of primers complementary to the denatured strands,
2) a DNA polymerase,
3) a DNA polymerase cofactor, and
4) a deoxyribonucleotide-5'-triphosphate, provided at least one of the PCR reagents 1) to 4) is encapsulated within an inert material which is a water-impermeable solid at room temperature, but which melts at a temperature of at least 50°C,

forming primer extension products of the primers and the denatured strands at a temperature of at least 50°C, and

C. separating the strands of the primer extension products, and
D. detecting at least one of the primer extension product strands as an indication of the presence of the target nucleic acid.

Still further, a method for controlling the formation of nonspecific nucleic acids during the amplification of a target nucleic acid by polymerase chain reaction, comprises:

amplifying a target nucleic acid in the presence of the following PCR reagents:

1) a primer complementary to the target nucleic acid,
2) a DNA polymerase,
3) a DNA polymerase cofactor, and
4) a deoxyribonucleotide-5'-triphosphate, provided at least one of the PCR reagents 1) to 4) is encapsulated within an inert material which is a water-impermeable solid at room temperature, but which melts at a temperature of at least 50°C.

The present invention overcomes the problem of amplification of non-target nucleic acids by keeping an essential PCR reagent needed for PCR isolated by encapsulation from other reagents until such time as PCR can proceed without the non-target nucleic acids being amplified. The formation of primer dimers is also greatly reduced. For example, any primed non-target nucleic acid can be heated to a temperature sufficient to dissociate the primers, and at the same time the encapsulated PCR reagent can be released to effect amplification of the target nucleic acid. The PCR reagent is encapsulated by a material which is inert to the reagent and other reagents in the PCR process. It is also a water-impermeable solid at room temperature, but melts at a temperature of at least 50°C. The improvements provided by this invention are more readily seen in the examples below.

Moreover, this invention avoids the problems associated with the use of a wax or grease barrier layer as described in WO-A-91/12342.

The general principles and conditions for amplification and detection of nucleic acids using polymerase chain reaction are quite well known, the details of which are provided in numerous references including US-A-4,683,195, US-A-4,683,202, US-A-4,965,188 and WO-A-91/12342 and by Guatelli and others, Clin.Microbiol.Rev., 2(2), pp. 217-226 (1989). In view of the teaching in the art and the specific teaching provided herein, a worker skilled in the art should have no difficulty in practicing the present invention by making the adjustments taught herein to accomplish amplification of a target nucleic acid with reduced amplification of non-target nucleic acids.

The present invention is directed to the amplification or detection of one or more specific nucleic acid sequences present in one or more target nucleic acids in a test specimen. Such specimens can include cellular or viral material, hair, body fluids or other materials containing genetic DNA or RNA which can be detected. While the primary purpose of detection is diagnostic in nature, the invention can also be used to improve the efficiency of cloning DNA or messenger RNA, or for obtaining large amounts of the desired sequence from a mixture of nucleic acids resulting from chemical synthesis.

The present invention is especially useful for producing, in exponential quantities relative to the number of reaction steps involved, at least one specific nucleic acid sequence in a nucleic acid associated with an infectious agent. The product will be a discrete nucleic acid duplex with termini corresponding to the ends of the specific primers employed. Any source of nucleic acid, purified or not, can be utilized as the starting material if it is known to or suspected of containing the specific nucleic acid sequence targeted for detection. Moreover, a plurality of target nucleic acids can be amplified and detected simultaneously by using a corresponding set of primers and detection means for each specific nucleic acid. Multiple sequences in the same nucleic acid can also be amplified and detected.

Nucleic acids to be detected can be obtained from various sources including plasmids, naturally occurring DNA or RNA from any source (such as bacteria, yeast, viruses, plants and higher animals, humans). It may be extracted from various tissues including blood, peripheral blood mononuclear cells (PBMC), tissue material or other sources known in the art using known procedures. The present invention is particularly useful for the amplification and detection of nucleic acid sequences found in genomic DNA, bacterial DNA, fungal DNA, viral RNA, or DNA or RNA found in bacterial or viral infected cells.

The method described herein can be used to provide the detection or characterization of specific nucleic acid sequences associated with infectious diseases, genetic disorders or cellular disorders such as cancers. It may also be used in forensic investigations and DNA typing. For purposes of this invention, genetic diseases include specific deletions or mutations in genomic DNA from any organism, such as sickle cell anemia, cystic fibrosis, α-thalassemia, β-thalessemia and others readily apparent to one skilled in the art. Human Leukocyte Antigen (HLA) can be categorized with the present invention. Bacteria which can be detected include, but are not limited to, Salmonella, Streptococcal organisms, Chlamydial organisms, Gonococcal organisms, Mycobacterium tuberculosis, Mycobacterium avium complex, Mycoplasma Haemophilus influenzae, Shigella and Listeria. Viruses which are detectable include, but are not limited to, herpes, Epstein Barr virus, cytomegalovirus, human papilloma virus, hepatitis and retroviruses such as HTLV-I, HIV-I and HIV-II. Protozoan parasites, yeasts and molds are also detectable. Other detectable species would be readily apparent to one skilled in the art. The invention is particularly useful for the detection of the presence of DNA associated with various bacteria or viruses, with the amplification and detection of viral DNA being of most interest. Detection of DNA associated with HIV-I (and other retroviruses), cytomegalovirus or human papilloma virus is advantageously accomplished with this invention. Most preferably, it is used to detect DNA associated with retroviruses, such as HIV-I.

As used herein in referring to primers, probes or oligomer fragments to be detected, the term "oligonucleotide" refers to a molecule comprised of two or more deoxyribonucleotides or ribonucleotides, and preferably more than three. Its exact size is not critical but depends upon many factors including the ultimate use or function of the oligonucleotide. The oligonucleotide may be derived synthetically, by cloning or by other methods known in the art.

A "PCR reagent" refers to any of the reagents considered essential to PCR, namely one or more primers for the target nucleic acid, a DNA polymerase, a DNA polymerase cofactor, and one or more deoxyribonucleotide-5'-triphosphates.

The term "primer" refers to an oligonucleotide, whether naturally occurring or synthetically produced, which is capable of acting as a point of initiation of synthesis when placed under conditions in which

4

synthesis of a primer extension product complementary to a nucleic acid strand (that is, template) is induced. Such conditions include the presence of nucleotides (such as the four standard deoxyribonucleoside-5'-triphosphates) a DNA polymerase, and suitable temperature, pH and cofactor.

The primer is preferably a single stranded oligodeoxyribonucleotide. It must be long enough to prime the synthesis of extension products in the presence of the DNA polymerase. The exact size of each primer will vary depending upon the use contemplated, the complexity of the targeted sequence, reaction temperature and the source of the primer. Generally, the primers used in this invention will have from 12 to 60 nucleotides, and preferably, they have from 18 to 45 nucleotides.

The primers used in the present invention are selected to be "substantially complementary" to the different strands of each specific sequence to be amplified. This means that they must be sufficiently complementary to hybridize with their respective strands to form the desired hybridized products and then be extendible by a DNA polymerase. In the preferred and most practical situation, the primer has exact complementarity to the target nucleic acid.

As used herein, a "probe" is an oligonucleotide which is substantially complementary to a nucleic acid sequence of the target nucleic acid and which is generally not able to form primer extension products. The probes can be of any suitable length of nucleotides, but generally, they have from 12 to 40 nucleotides. They are labeled (commonly at the 3' end) with any suitable detectable material (either directly or indirectly), as described below. They can also be attached to a water-insoluble substrate of some type for capture of the target nucleic acid using known technology.

A DNA polymerase is an enzyme which will add deoxynucleoside monophosphate molecules to the 3' hydroxy end of the primer in a complex of primer and template, but this addition is in a template dependent manner (that is, dependent upon the nucleotides in the template).

Preferably, the invention is carried out using a "thermostable DNA polymerase" which is stable to heat, especially the high temperatures used for denaturation of DNA strands. More particularly, the thermostable DNA polymerases are not substantially inactivated at the high temperatures used in polymerase chain reactions as described herein. Such temperatures will vary depending upon a number of reaction conditions, including pH, the nucleotide composition of the target nucleic acid and primers, the length of primer, salt concentration and other conditions known in the art and will be in the ranges noted below.

A number of thermostable DNA polymerases have been reported in the art, including those mentioned in detail in US-A-4,965,188 and US-A-4,889,818. Particularly useful polymerases are those obtained from various Thermus bacterial species, such as Thermus aquaticus, Thermus thermophilus or Thermus flavus. Other useful thermostable polymerases are described in WO-A-89/06691.

A DNA polymerase cofactor refers to a non-protein compound on which the enzyme depends for activity. Thus, the enzyme is catalytically inactive without the presence of the cofactor. The exact mechanism of the interaction of the cofactor with the DNA polymerase is unknown at present. A number of such materials are known cofactors including manganese and magnesium compounds. Such compounds contain the manganese or magnesium in such a form that divalent cations are released into an aqueous solution under conditions of pH 6 to 9 and temperatures over 50°C. Useful cofactors include, but are not limited to, manganese and magnesium salts, such as chlorides, sulfates, acetates and fatty acid salts (for example, butyric, caproic, caprylic, capric and lauric acid salts). The smaller salts, that is chlorides, sulfates and acetates, are preferred.

Magnesium salts, such as magnesium chlorides and sulfates, are most preferred in the practice of the invention.

Also needed for PCR is a deoxyribonucleotide-5'-triphosphate, such as dATP, dCTP, dGTP or dTTP. Usually, all four are used in PCR. Analogues such as dITP and 7-deaza-dGTP are also useful.

The material used for encapsulating a PCR reagent in the practice of this invention is "inert" to the PCR process, meaning that it does not participate in or adversely affect the operation of PCR except to keep the encapsulated reagent from reaction until released. The material is a water-impermeable solid at room temperature (generally from 15 to 30°C) meaning that no water passes through it until the material melts generally at a temperature of at least 50°C. Preferably, the encapsulating material melts at a temperature of at least 50°C in a very short time (that is, a few seconds).

Useful encapsulating materials include, but are not limited to, waxes, singly or in admixture. A wax is an organic substance (synthetically prepared or naturally occurring, such as mineral, vegetable or animal waxes) which melts at or above 50°C to form a liquid having a density lower than that of water. Usually, waxes are esters of high molecular weight fatty acids with a high molecular weight alcohol. Representative pure waxes include eicosane, octacosane, cetyl palmitate and pentaerythritol tetrabehenate. Many useful waxes are mixtures of materials (such as esters, fatty acids, high molecular weight alcohols and hydrocarbons) which provide properties similar to those of pure waxes. Such mixtures include, but are not limited to, paraffin, PARAPLAST™ wax (Sherwood Medical), ULTRAFLEX™ wax (Petrolite Corporation) and BESQUARE™ 175 wax (Petrolite Corporation). Waxes can be purchased or prepared by mixing pure or mixed waxes with one another or with suitable greases or oils which preserve the relative hardness, reduced stickiness typical of waxes, and desired melting temperatures.

The oils and greases described in WO 91/12342 (noted above) are not useful as encapsulating materials.

A preferred group of encapsulating materials include mixed waxes, such as paraffin.

The art of microencapsulation has been developing for some time and is accomplished, in general, by physical, chemical or mechanical methods, or combinations thereof. More specifically, useful procedures include, but are not limited to, coacervation (including complex coacervation), phase separation, dehydration (by atomization or using an organic solvent or polymer), spray drying, fluidized bed, chemical vapor deposition, pan coating, *in situ* polymerization (when coating with polymers), interfacial polymerization, centrifugally forcing core material through coating films or projecting them through hollow tubes through a curtain of coating materials, electrostatic processes and extrusion processes.

The details of such encapsulation methods are well known in the art, including such texts as those by Vandegaer (Ed.), Microencapsulation Processes and Applications, Plenum Press, New York, 1974; Gutcho, Microcapsules and Microencapsulation Techniques, Noyes Data Corporation, Park Ridge, New Jersey, 1976; and Kondo, Microcapsule Processing and Technology, Marcel Dekker, Inc., New York, 1979.

In general, microcapsules have an average diameter in the range of from 0.1 to 25 mm, while diameters of from 1 to 10 mm are more common. The thickness of microcapsule walls generally range from 0.2 to 100 μmeters.

Representative methods for encapsulating PCR reagents are described in Examples 1 and 2 below.

Preferably, the PCR coreagents and triphosphates are encapsulated in the practice of this invention. More particularly, the encapsulated reagent is a magnesium salt, manganese salt or a deoxyribonucleotide triphosphate.

The encapsulated PCR reagent can be supplied as an individually packaged component of a diagnostic test kit having individually packaged reagents and equipment useful for carrying out PCR, as described herein. Alternatively, the encapsulated PCR reagent can be included in a composition along with one or more other PCR reagents.

The PCR reagents are provided and used in PCR in any concentration suitable for a given process. The minimal amounts needed for amplification and suitable ranges of concentrations are well known in the art. The reagents can be used together in any combination or added sequentially.

A target nucleic acid (that is, one to be amplified or detected) can be obtained from any of a variety of sources as noted above. Generally, it is extracted in some manner to make it available for contact with the primers and other PCR reagents. This usually means removing unwanted proteins and cellular matter from the specimen in a suitable manner. Various procedures are known in the art, including those described by Laure and others in The Lancet, pp. 538-540 (Sept. 3, 1988), Maniatis and others, Molecular Cloning: A Laboratory Manual, pp. 280-281 (1982), Gross-Belland and others in Eur.J.Biochem., 36, 32 (1973) and US-A-4,965,188. Extraction of DNA from whole blood or components thereof are described, for example, in EP-A-0 393 744, Bell and others, Proc. Natl. Acad. Sci. USA, 78(9), pp. 5759-5763 (1981) and Saiki and others, Bio/Technology, 3, pp. 1008-1012 (1985).

Since the nucleic acid to be amplified or detected is usually in double stranded form, the two strands must be separated (that is, denatured) before priming can take place. This can occur during the extraction process, or be a separate step afterwards. Denaturation is accomplished using a heat treatment alone or in combination with any suitable other physical, chemical or enzymatic means as described in the art. Denaturation is generally carried out by heating the specimen suspected of containing the targeted nucleic acid at a first temperature of from 85 to 100°C for a suitable time, for example from 1 second to 3 minutes.

The denatured strands are then cooled to a temperature which is generally in the range of from 55 to 70°C. The time needed for cooling the denatured strands will vary depending upon the type of apparatus used for the PCR process.

Once the denatured strands are cooled to the second temperature, the reaction mixture containing PCR reagents is incubated at a suitable temperature to effect formation of primer extension products. Generally, this temperature is at least 50°C, and preferably in the range of from 55 to 70°C. This incubation is effective to maintain the melted state of the encapsulating material and to allow the encapsulated PCR reagent to participate in the PCR reaction. The time for incubation can vary widely depending upon the incubation temperature, but in preferred embodiments, it is from 1 to 90 seconds.

The primer extension products thus formed can be detected in a suitable manner while as hybridized products, or denatured either for detection of a single strand or further cycling in PCR.

If the hybridized primer extension products are denatured, PCR can be carried out further in as many cycles of priming, extension and denaturation as desired. Generally, at least 20 cycles will be carried out, with from 20 to 50 cycles being preferred.

After denaturation the last time in the assay, the final primer extension products can be detected using known procedures, as described below. Alternatively, the primer extension products can be detected in undenatured form using known procedures such as agarose gel electrophoresis, ethidium bromide staining or both.

The amplification method of this invention is preferably conducted in a continuous, automated manner so that the reaction mixture is temperature cycled in a controlled manner for desired preset times. A number of instruments have been developed for this purpose, as one of ordinary skill in the art would know. Such instruments are commercially available from Perkin Elmer/Cetus.

One instrument utilizes temperature cycling without a liquid handling system, and is described in some detail in US-A-4,965,188 and EP-A-0 236 069. Generally, this instrument includes a heat conducting container for holding a number of reaction tubes containing reaction mixture, a means for heating, cooling and temperature maintenance, and a computing means to generate signals to control the amplification sequence, changes in temperature and timing.

An instrument for processing amplification reactions in a disposable chemical test pack is described in some detail in EP-A-0 402,994. In general, this instrument comprises a surface for supporting a chemical test pack, pressure applicators supported above the surface for acting on the reaction pack to transfer fluids between adjacent chambers in the test pack, and means for operating the pressure applicators through a range of movement extending across the test pack.

EP-A-0 402,994 provides details of useful chemical test packs which can be processed using the instrument described in that same publication. Also described therein are means for heating and cooling the test pack at repeated intervals (that is, through cycles) appropriate for the method of the present invention.

The method of this invention can be used to advantage to rapidly detect or characterize a targeted nucleic acid which is present in an infectious agent. Detection can be accomplished in a number of known ways, such as those described in US-A-4,965,188. For example, the amplified nucleic acid can be analyzed using Southern blotting techniques. Alternatively, amplification can be carried out using radioisotopic or biotinylated primers which can then be detected using appropriate techniques. Sequence specific oligonucleotides can be used with dot blot techniques to detect single-base pair variations in nucleic acids.

In one preferred embodiment, once a desired amount of the targeted nucleic acid of interest has been generated and the primer extension products are denatured for a last time, the amplified targeted nucleic acid is detected using an oligonucleotide probe which is labeled for detection and can be directly or indirectly hybridized with one of the primer extension products. Procedures for attaching labels and preparing probes are well known in the art, for example, as described by Agrawal and others, Nucleic Acid Res., 14, pp. 6227-45 (1986), US-A-4,914,210 (Levenson and others) relating to biotin labels, US-A-4,962,029 relating to enzyme labels, and the references noted therein. Useful labels include radioisotopes, electron-dense reagents, chromogens, fluorogens, phosphorescent moieties, ferritin and other magnetic particles (see US-A-4,795,698 and US-A-4,920,061), chemiluminescent moieties and enzymes (which are preferred). Useful enzymes include, glucose oxidase, peroxidases, uricase, alkaline phosphatase and others known in the art and can be attached to oligonucleotides using known procedures. Substrates and dye forming compositions for such enzymes are well known.

Where the label is a preferred enzyme such as a peroxidase, at some point in the assay, hydrogen peroxide and suitable dye-forming compositions are added to provide a detectable dye. For example, useful dye-providing reagents include tetramethylbenzidine and derivatives thereof, and leuco dyes, such as triarylimidazole leuco dyes (as described in US-A-4,089,747), or other compounds which react to provide a dye in the presence of peroxidase and hydrogen peroxide. Particularly useful dye-providing compositions are described in EP-A-0 308 236.

Detection of the presence of the probe which is in the complementary product can be achieved using suitable detection equipment and procedures which are well known. Certain probes may be visible to the eye without the use of detection equipment.

In a preferred embodiment, one of the primers is biotinylated and the amplified nucleic acid is detected using detectably labeled avidin or a derivative thereof. For example, avidin can be conjugated preferably with an enzyme, or have a radioactive moiety. Biotin on the amplified product complexes with the avidin, and appropriate detection techniques are used.

Other formats for detection are well known in the art which includes standard hybridization procedures (such as "sandwich assays), and other procedures described in the amplification art such as US-A-4,965,188.

It is also useful for the method of this invention to be carried out in a suitable container, such as a test tube, cuvette, flask or beaker, or more sophisticated containers which have been fashioned to facilitate automated procedures (see for example, WO-A-91/12342). For example, cuvettes constructed to provide certain temperature characteristics during the practice of the method are described in US-A-4,902,624 and EP-A-0 381,501. Such containers are also known as chemical test packs (or pouches) as described above. Such test packs have a multiplicity of reaction chambers having various reagents, buffers and other materials which are useful at various stages in the amplification and detection methods. The packs can be appropriately and rapidly heated and cooled in cycles to promote the various steps of the amplification method of this invention. Other useful containers could be suitably fashioned for the method of this invention.

In order for the amplified product to be detected, it is often useful (but not necessary) for it to be separated from the other materials in the reaction medium. This is done by any of a number of ways, including using a water-insoluble capture means on a primer or probe so that the primer extension products which are replicated in the method are water-insolubilized and removed from the reagent mixture. Primers or probes can be attached to insoluble materials in a suitable manner, or they can be designed to be capturable, that is, reactive with a capture means at some point in the method.

One useful capture means is described in EP-A-0 370 694. A primer has a specific binding ligand attached thereto (such as biotin, an antibody or a lectin) which is capable of specifically binding to a receptor molecule (such as avidin, an antigenic material or a sugar) which is bound in a suitable manner to an insoluble material such as polymeric particles. The resulting insolubilized specifically bound product can be separated from water-soluble materials by filtration, centrifugation or other suitable separation techniques. Detection of the captured nucleic acid strand can be accomplished directly using a probe complementary thereto, or indirectly using one or more intermediate oligonucleotides to which a labeled probe can be hybridized.

Alternatively, the amplified product can be separated from undesired materials by using an oligonucleotide complementary thereto, which oligonucleotide is attached to an insoluble substrate (such as polymeric particles) using known attachment techniques. One such technique is described in EP-A-0 439,222. Other techniques are described for example in US-A-4,713,326, WO-A-88/01302 and EP-B-0 070 687 so that intermediate oligonucleotides are used in a hybridized product of multiple components to which the capture oligonucleotide and amplified nucleic acid are joined.

Useful separation means are microporous filtration membranes such as the polyamide membranes marketed by Pall Corp. (for example as LOPRODYNE™ or BIODYNE™ membranes). They can be used uncoated or precoated with surfactants or other materials which facilitate the analytical procedures.

The membranes can be used as a separate substrate with suitable containers for carrying out other steps of the assay. Preferably, however, they are mounted as part of a disposable test device. Various disposable test devices are known in the art including those described in US-A-3,825,410, US-A-3,888,629, US-A-3,970,429 and US-A-4,446,232. Particularly useful devices are described in US-A-4,921,677 and are commercially available as SURECELL™ test devices and assay kits from Eastman Kodak Company.

Any useful solid support can be used for separation of water-insoluble product for detection, including a microtiter plate, test tube, beaker, beads, film, membrane filters, filter papers, gels, magnetic particles or glass wool. It can be made of a number of materials including glass, ceramics, metals, naturally occurring or synthetic polymers, cellulosic materials, filter materials and others readily apparent to one of ordinary skill in the art. Particularly useful solid support materials are polymeric beads generally having an average particle size of from 0.1 to 10 $\mu$meters.

The detection can also be carried out by immobilizing a capture probe on a flat substrate, such as the microporous filtration membranes described above, or on thin polymeric films, uncoated papers or polymer coated papers, a number of which are known in the art. Other details about such materials are provided in EP-A-0 408 738.

Although this disclosure has focused on the use of the claimed composition and test kit in PCR, the invention is also useful in other procedures for enzymatic replication of nucleic acids, such as the transcription based amplification technique described by Kwoh and others, Proc.Natl.Acad.Sci.USA 87:1974, 1989, nucleic acid ligase techniques described by Wu and others, Genomics 4:560, 1989 and Barringer and others, Gene 89:117, 1990, and ribonuclease H cleavage of DNA-RNA-DNA probes annealed to nucleic acid targets.

The following examples are included to illustrate the practice of this invention, and are not meant to be limiting in any way. All percentages are by weight unless otherwise noted.

Example 1 PCR Reagent Composition Having Encapsulated Magnesium Compound

This example illustrates the preparation of a PCR reagent composition having an encapsulated magnesium salt cofactor. It illustrates a procedure for encapsulating the magnesium salt cofactor using paraffin.

A magnesium salt was encapsulated by heating paraffin (3.7 g) to 75-80°C until it was completely melted. Anhydrous magnesium chloride (0.3 g) was then stirred into the melted paraffin until a uniform suspension was formed. The suspension was slowly cooled until it became tacky, and it was maintained in this form. Small portions of the tacky suspension were removed and formed into spheres having a diameter of about 5 mm. These spheres were then cooled to room temperature, washed in cold water to remove any magnesium chloride on the surface, dried and stored at room temperature. Each sphere contained about 5.9-6.7% of magnesium chloride cofactor.

This encapsulated PCR reagent was tested for water impermeability by suspending it overnight at room temperature in sufficient water to provide 100 mmolar of magnesium ion if all of the salt was released. An aliquot (2.5%) of the water was removed from the suspension, replaced with fresh water, and the remaining suspension was heated for 5 minutes at 74°C to melt the paraffin and release the magnesium chloride. A similar aliquot of fluid was removed after this heating step.

The amount of magnesium chloride in each aliquot was determined by measuring DNA polymerase activity. DNA polymerase activity was measured as follows:

Enzyme from Thermus aquaticus was assayed at 2.4 units/ml in a solution of N-tris(hydroxymethyl)-methyl-3-aminopropanesulfonic acid buffer (25 mmolar, pH 9.8), potassium chloride (50 mmolar), 2-mercaptoethanol (1 mmolar), activated salmon testes DNA (0.29 $\mu$g/$\mu$l), dCTP, dGTP and dTTP (200 $\mu$molar of each) and $^3$H-dATP (100 $\mu$molar, 0.02 $\mu$Ci/$\mu$l). Each assay was carried out using one of the following as a source of magnesium ion: either one of the aqueous suspensions noted above, a stock solution of magnesium chloride (100 mmolar) as a positive control, or water as a negative control. The DNA polymerase activity observed in each reaction mixture was sensitive to the level of free magnesium ion present. The DNA polymerase is inactive with 0.25 mmolar magnesium ion and has optimum activity with about 2 mmolar magnesium ion.

The data from these tests are presented below in Table I in terms of units/ml of DNA polymerase activity and "percent of Control" represented by reaction in the presence of the stock solution. It is apparent that the magnesium chloride coreagent was completely inaccessible for reaction prior to heating the encapsulated material, and was completely available thereafter.

## TABLE I

| Aliquot | Enzyme Activity (Units/ml) | % of Control |
|---|---|---|
| Stock Mg$^{+2}$ (control) | 2.81 | 100 |
| No enzyme | 0.01 | 0.3 |
| Water only | 0 | 0 |
| Encapsulated Mg$^{+2}$:* | | |
|    Before heating | 0.02, 0 | 0.5, 0 |
|    After heating | 2.91, 2.89 | 103.6, 103.0 |

*The results from two samples.

These data indicate that encapsulated magnesium chloride would be useful to prevent nonspecific amplification during PCR processes.

Example 2 PCR Reagent Having Encapsulated Nucleoside Triphosphates

This example is similar to Example 1 except that the four dNTP's were encapsulated instead of the magnesium chloride cofactor.

The encapsulation process was as follows:

Paraffin (0.5 g) was heated to 75-80°C and maintained at that temperature until it was completely melted. The nucleoside triphosphates dATP, dCTP, dGTP and dTTP (2 mg of each) were added to the melted paraffin with stirring to form a uniform suspension. While keeping the suspension at 75-80°C, it was taken up with a PASTEUR™ pipette and transferred dropwise to water being held at about 0-4°C. The drops became solidified particles in the water, each containing about 10 mg of paraffin and encapsulated reagents. Upon drying, the oval-shaped particles had dimensions of about 3 mm in diameter and 1-2 mm in height and were stored at -20°C.

Example 3 PCR Assay Using Encapsulated Cofactor

This example demonstrates the use of the PCR reagent composition described in Example 1 in a PCR method to detect a targeted nucleic acid, namely HIV-I DNA incorporated at a single-copy level in a human cell line, identified in the art as HUT AAV78 DNA, present at a concentration of about 1000 copies of HIV-I/100 $\mu$l.

Four PCR reagent solutions were prepared containing tris(hydroxymethyl)aminomethane buffer (10 mmolar, pH 8), potassium chloride (50 mmolar), gelatin (0.1 mg/ml), primers (identified below, 1 $\mu$molar of each), four standard dNTP's (6 mmolar total, 1.5 mmolar of each), human placental DNA (2 $\mu$g) and DNA polymerase from Thermus aquaticus (32 units in 200 $\mu$l total volume). Each solution was put into six different reaction tubes (0.2 ml volume). In addition, these solutions contained the following:

Solution 1 contained magnesium chloride (10 mmolar) free in solution, and no target nucleic acid.

Solution 2 contained magnesium chloride (10 mmolar) free in solution along with 2000 copies of target nucleic acid per 200 $\mu$l.

Solution 3 contained paraffin particles (without magnesium chloride) with 2000 copies of target nucleic acid per 200 $\mu$l.

Solution 4 contained encapsulated magnesium chloride (sufficient to release 10 mmolar cofactor) with 2000 copies of target nucleic acid per 200 $\mu$l.

Three tubes of each solution were used in PCR immediately after the solutions were prepared, while the remaining tubes were kept for five hours at 20-25°C after solution preparation, and then used in PCR.

The primers had the following sequences and were obtained by synthesis on an Applied Biosystems Model 380B DNA synthesizer:

SEQ ID NO:1:

5'-ATAATCCACC TATCCCAGTA GGAGAAAT-3'

SEQ ID NO:2:

5'-X-TTTGGTCCTT GTCTTATGTC CAGAATGC-3'

SEQ ID NO:1: and SEQ ID NO:2: are well known in the art as "SK38" and "SK39", respectively, and "X" represents a biotin moiety attached to the sequence through two tetraethylene glycol spacer units.

The following PCR protocol was carried out using a Perkin-Elmer thermocycling instrument:

Denaturing at 95°C for about 15 seconds,

Cooling to 55°C over 30 seconds, and

Formation of primer extension products at 70°C for about 30 seconds.

After the last cycle, the amplified targeted nucleic acid was detected using standard gel electrophoresis/ethidium bromide staining.

For the reagent solutions used in PCR immediately after mixing, both solutions 2 and 4 gave specific product. However, the specific product band was stronger with solution 4, and the gel lane contained no nonspecific PCR products. The gel lane with solution 2 contained considerable nonspecific products from PCR. Also, solution 2 produced some primer/dimer product whereas solution 4 did not. The gel lane for solution 3 was clear.

For the solutions used in PCR 5 hours after mixing, solutions 1 and 2 produced an extensive range of nonspecific products as well as targeted product. The gel lane for solution 3 was clear, and the gel lane for solution 4 showed a distinct band for targeted nucleic acid with no observable nonspecific product or primer dimer products.

These results indicate that the encapsulated PCR reagent provided the desired amplification and detection of a targeted nucleic acid with little or no formation of nonspecific amplified products or primer/dimer product.

Example 4 PCR Carried Out Using Test Pack

This example demonstrates the use of an encapsulated cofactor in PCR carried out using a test pack for detection of product, such as the PCR product described in Example 3. Solutions 1-4 described in Example 3 were tested and the results are provided in Table II below.

The capture probe used in this example was prepared as follows:

Polymeric particles (1.2 $\mu$m average diameter) of poly[styrene-co-mono-2-(p-vinylbenzylthio)ethyl succinate] (95:5 weight ratio) were prepared using known emulsion polymerization techniques. To these particles were attached molecules of the oligonucleotide having the sequence:

SEQ ID NO:3:

5'-Y-ATCCTGGGAT TAAATAAAAT AGTAAGAATG TATAGCCCTA C-3'

wherein "Y" is an aminediol linking group with two tetraethylene glycol spacer groups prepared and attached to the oligonucleotide according to the teaching of US-A-4,962,029 (noted above).

The particles were washed twice with 2-(N-morpholino)ethanesulfonic acid buffer (0.1 molar, pH 6). Samples (30 $\mu$g) of the particles suspended in the buffer (1 ml) were mixed with 1-(3-dimethylaminopropyl)-3-ethylcarbodiimide hydrochloride (0.15 ml of 100 mg/ml of buffer) and the oligonucleotide (0.0288 ml of 57.3 OD/ml of purified water, 1.65 OD units or 55 $\mu$g). The mixture was rotated end over end at room temperature (20-25°C) for 15 hours and centrifuged. The particles were washed three times with water and resuspended in purified water at a solids content of 0.9%.

The capture probe was mixed with an adhesive of poly(methyl acrylate-co-2-acrylamido-2-methylpropanesulfonic acid, sodium salt-co-2-acetoacetoxyethyl methacrylate) (90:4:6 weight ratio). The resulting composition comprised about 18% (dry weight) of adhesive.

A conjugate of streptavidin and horseradish peroxidase was obtained from Zymed Labs (San Francisco) and was diluted 1:8000 with casein (0.5%) in a phosphate buffer solution (pH 7.3) containing thimerosal preservative (0.01%).

A leuco dye composition was prepared as follows: 2-(4-hydroxy-3,5-dimethyoxyphenyl)-4,5-bis(4-methoxyphenyl)imidazole (to make a 0.1% solution) was dissolved in a solution of poly(vinylpyrrolidone) (20%) in sodium phosphate buffer (5 mmolar). This solution was then added to a solution containing hydrogen peroxide (10 mmolar), 4'-hydroxyacetanilide electron transfer agent (5 mmolar) and diethylenetriaminepentaacetic acid chelating agent (10 $\mu$molar) in sodium phosphate buffer to produce a final concentration of 1% poly(vinyl pyrrolidone) and 0.005% leuco dye.

The test packs were prepared by heating a sheet of poly(ethylene terephthalate)/polyethylene laminate (SCOTCHPAK™ 241, 3M Co.) at a forming station (or mold) to form an array of depressed areas toward one side of the sheet, so that upon lamination to a cover sheet at a later time, the resulting pouch had narrow channels leading from the depressed areas to a main channel. Each depressed area was later filled with an appropriate composition or solution. A sheet was laminated to form a cover over the depressed and channel areas, and sealed to create a burst seal between each depressed area and the channel leading from it to the main channel. First, however, one section near the end of the main channel was treated with corona discharge from an Enercon DYNE-A-MITE™ corona discharge unit at 0.95 cm clearance to a TEFLON™ dielectric plate for 8 seconds. The capture probe was then immediately deposited as a spot on the treated surface, each spot having 0.9 to 1.1 $\mu$l of the formulation. The disposed formulation was dried for about 20 seconds in a stream of air at room temperature while heating the opposite side of the support with an iron at about 95°C.

The blisters (or chambers) formed in the test pack were filled with the desired reagents. A first blister of each test pack was adapted to receive a sample of one of Solutions 1-4 to be tested after PCR amplification which was performed in tubes (like Example 3).

A second blister was filled with a streptavidin-horseradish peroxidase conjugate composition as described above. A third blister contained a 1% sodium decylsulfate wash solution (in phosphate buffered saline solution containing 10 mmolar sodium phosphate, 150 mmolar sodium chloride and 1 mmolar ethylenediaminetetraacetic acid, pH 7.4), and a fourth blister contained the leuco dye composition (described above). A last blister located at the end of the main channel was larger than the others and fitted with an absorbent to be a reservoir for waste fluids.

The cover sheet was then laminated and sealed in three steps. First, the sandwich was pressed and sealed by heating at about 149°C only around the blisters containing the 3 reagent solutions and around the waste blister. The formation of the sample-receiving PCR blister, including burst seals, and the channels was completed by heating the test pack between appropriately shaped heating jaws at about 163°C. The third step was the formation of perimeter seals around the test pack, and resealing all blister perimeter seals using a top plate temperature of 199°C while the bottom plate remained at ambient temperature. The channels formed in the completed test pack were located so that passage of a roller across the portion of the test pack containing the blisters would sequentially burst the seals of the blisters and force the reagent from each blister into and along an exit channel to the main channel leading to the area containing the capture probes.

The completed integral test packs were used to evaluate Solutions 1-4 as follows:

The PCR products from Solutions 1-4 (described in Example 3) were diluted 20 times in a buffer containing tris(hydroxymethyl)aminomethane buffer (10 mmolar, pH 8), magnesium chloride (10 mmolar), potassium chloride (50 mmolar) and gelatin (0.1 mg/ml). About 200 $\mu$l of the diluted product was introduced into the first blister of the test pack by using a 1 ml disposable tuberculin syringe, and the blister was sealed. The blister containing one of Solutions 1-4 was preheated to 95-98°C for 120 seconds and then rolled to break the seal and advance the solution to the area containing the capture probe. The target nucleic acid (if present) and capture probe were hybridized in the detection blister during incubation at 42°C for 5 minutes. The seal of the conjugate blister was broken, and the solution directed to the detection area, where it complexed with the available amplified biotinylated target nucleic acid for five minutes. The seal of the wash solution blister (preheated to 55°C) was then broken and the wash solution directed to the detection area to clean out the main channel and to remove unbound conjugate from the detection area. The seal of the blister containing the leuco dye was then broken and its contents were directed to the detection area. Incubation for five minutes allowed dye formation to occur before the color density scores were read.

The color density scores, provided in Table II below, are visual observations with "0" representing no density and "10" representing the highest density.

The tests labeled with "i" were carried out immediately after the reagents were mixed, whereas those labeled with "ℓ" were carried out after incubation of the reagents for five hours at 22°C.

TABLE II

| Test Solution | Dye Density | |
|---|---|---|
| | Assay | Background |
| 4"i" | 5 | 0 |
| 2"i" | 6.5 | 0 |
| 3"i" | 1.5 | 1.5 |
| 1"i" | 0.5 | 0 |
| 4"i" | 3 | 1 |
| 3"i" | 1 | 1 |
| 2"i" | 7 | 1 |
| 1"i" | 1 | 1 |
| 1"i" | 1 | 1 |
| 2"i" | 7.5 | 0 |
| 3"i" | 1 | 1 |
| 4"i" | 6.5 | 0 |
| 4"ℓ" | 7 | 0.5 |
| 3"ℓ" | 1 | 0.5 |
| 2"ℓ" | 4.5 | 0 |
| 1"ℓ" | 0 | 0 |
| 1"ℓ" | 1 | 1 |
| 2"ℓ" | 6 | 0.5 |
| 3"ℓ" | 1 | 1 |
| 4"ℓ" | 8 | 1 |
| 4"ℓ" | 1 | 1 |
| 3"ℓ" | 0 | 0 |
| 2"ℓ" | 5 | 0 |
| 1"ℓ" | 0 | 0 |

**Claims**

1. A PCR reagent composition comprising a PCR reagent, the composition characterized wherein the PCR reagent is encapsulated within an inert material which is a water-impermeable solid at room temperature, but which melts at a temperature of at least 50°C.

2. The composition as claimed in claim 1 wherein the encapsulated PCR reagent is a magnesium salt, a manganese salt or a deoxyribonucleotide-5'-triphosphate.

3. The composition as claimed in either of claims 1 or 2 wherein the encapsulated PCR reagent is encapsulated with a wax.

4. A diagnostic test kit for polymerase chain reaction comprising, individually packaged, two or more PCR reagents, the kit characterized wherein at least one of the PCR reagents is encapsulated within an inert material which is a water-impermeable solid at room temperature, but which melts at a temperature of at least 50°C.

5. The test kit as claimed in claim 4 wherein the encapsulated PCR reagent is:
   a. a primer,
   b. a DNA polymerase,
   c. a DNA polymerase cofactor, or
   d. a deoxyribonucleotide-5'-triphosphate.

6. A method for the amplification of a targeted nucleic acid comprising the steps of:
   A. contacting a specimen suspected of containing a targeted nucleic acid with the following PCR reagents, in any order,
   1) a primer complementary to the targeted nucleic acid,
   2) a DNA polymerase,
   3) a DNA polymerase cofactor, and
   4) a deoxyribonucleotide-5'-triphosphate, provided at least one of the PCR reagents 1) to 4) is encapsulated within an inert material which is a water-impermeable solid at room temperature, but which melts at a temperature of at least 50°C, and
   B. heating the resulting mixture of the specimen and the encapsulated PCR reagent at a temperature sufficient to melt the inert encapsulating material and to form a primer extension product, the temperature being at least 50°C.

7. The method as claimed in claim 6 wherein the specimen is contacted with all of the PCR reagents simultaneously.

8. The method as claimed in either of claims 6 or 7 wherein the DNA polymerase cofactor is encapsulated with a wax, and the DNA polymeraae cofactor is a magnesium salt.

9. A method for the amplification and detection of a targeted double-stranded nucleic acid comprising the steps of:
   A. heating a targeted double-stranded nucleic acid to denature its strands,
   B. in the presence of the following PCR reagents:
      1) a set of primers complementary to the denatured strands,
      2) a DNA polymerase,
      3) a DNA polymerase cofactor, and
      4) a deoxyribonucleotide-5'-triphosphate, provided at least one of the PCR reagents 1) to 4) is encapsulated within an inert material which is a water-impermeable solid at room temperature, but which melts at a temperature of at least about 50°C,
         forming primer extension products of the primers and the denatured strands at a temperature of at least 50°C, and
   C. separating the strands of primer extension products, and
   D. detecting at least one of the primer extension product strands as an indication of the presence of the targeted nucleic acid.

10. The method of claim 9 wherein the DNA polymerase cofactor or the deoxyribonucleotide-5'-triphosphate is encapsulated, and the DNA polymerase is a thermostable DNA polymerase isolated from a Thermus species or is an equivalent thereof prepared using recombinant technology.

11. The method of claim 9 wherein one of the primers is labeled with a specific binding capture ligand, and detection in step D is carried out by capturing the labeled primer by complexation with a receptor for the specific binding capture ligand which is immobilized on a solid substrate.

12. The method of claim 9 wherein the labeled primer is labeled with a specific binding detection ligand, and detection in step D is carried out by complexing the labeled primer with a detectably labeled receptor for the specific binding detection ligand.

13. The method of claim 12 wherein the specific binding detection ligand is biotin, and the detectably labeled receptor is detectably labeled avidin.

14. A method for controlling the formation of nonspecific nucleic acids during the amplification of a targeted nucleic acid by polymerase chain reaction, comprising:
      amplifying a targeted nucleic acid in the presence of the following PCR reagents:
      1) a primer complementary to the targeted nucleic acid,
      2) a DNA polymerase,
      3) a DNA polymerase cofactor, and
      4) a deoxyribonucleotide-5'-triphosphate, provided at least one of the PCR reagents 1) to 4) is encapsulated within an inert material which is a water-impermeable solid at room temperature, but which melts at a temperature of at least about 50°C.

European Patent Office

# EUROPEAN SEARCH REPORT

Application Number

EP 93 20 1281

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | WO-A-9 112 342 (CETUS CORP.)<br>* the whole document *<br>--- | 1-14 | C12Q1/68<br>C12Q1/70 |
| D,A | PHARMACEUTICAL RESEARCH<br>vol. 7, no. 11, 1990,<br>pages 1119 - 1126<br>M. J. JOZWIAKOWSKI 'Characterization of a hot-melt fluid bed coating process for fine granules'<br>* the whole document *<br>--- | 1-14 | |
| A | WO-A-9 118 110 (MALMQUIST)<br>--- | - | |
| D,A | BIOTECHNIQUES<br>vol. 13, no. 2, February 1992, NATICK, MA US<br>pages 266 - 274<br>G. RUANO ET AL. 'Heat-soaked PCR: an efficient method for DNA amplification with applications to forensic analysis'<br>--- | - | |
| E | WO-A-9 316 200 (K. KOSAK ET AL.)<br>* the whole document *<br><br>----- | 1-14 | TECHNICAL FIELDS SEARCHED (Int. Cl.5 )<br><br>C12Q |

The present search report has been drawn up for all claims

| Place of search | Date of completion of the search | Examiner |
|---|---|---|
| THE HAGUE | 13 SEPTEMBER 1993 | MOLINA GALAN E. |

<u>SEQUENCE LISTING</u>

INFORMATION FOR SEQ ID NO:1:

(i)   SEQUENCE CHARACTERISTICS:

    (A)   LENGTH:  28 nucleotides

    (B)   TYPE:  Nucleic acid

    (C)   STRANDEDNESS:  Single

    (D)   TOPOLOGY:  Linear

(ii) MOLECULE TYPE:  Primer

(iii) HYPOTHETICAL:  No

(iv) ANTI-SENSE:  No

(vi) ORIGINAL SOURCE:  Synthetically prepared

(vii) IMMEDIATE SOURCE:  Same

(x)   PUBLICATION INFORMATION:  US-A-4,965,188

(xi) SEQUENCE DESCRIPTION:  SEQ ID NO:1:

    ATAATCCACC TATCCCAGTA GGAGAAAT    28


INFORMATION FOR SEQ ID NO:2:

(i)   SEQUENCE CHARACTERISTICS:

    (A)   LENGTH:  28 nucleotides

    (B)   TYPE:  Nucleic acid

    (C)   STRANDEDNESS:  Single

    (D)   TOPOLOGY:  Linear

(ii) MOLECULE TYPE:  Primer

(iii) HYPOTHETICAL:  No

(iv) ANTI-SENSE:  No

(vi) ORIGINAL SOURCE:  Synthetically prepared

(vii) IMMEDIATE SOURCE:  Same

(x)   PUBLICATION INFORMATION:  US-A-4,965,188

(xi) SEQUENCE DESCRIPTION:  SEQ ID NO:2:

    TTTGGTCCTT GTCTTATGTC CAGAATGC    28


INFORMATION FOR SEQ ID NO:3:

(i)   SEQUENCE CHARACTERISTICS:

    (A)   LENGTH:  41 nucleotides

    (B)   TYPE:  Nucleic acid

    (C)   STRANDEDNESS:  Single

    (D)   TOPOLOGY:  Linear

(ii) MOLECULE TYPE:  Capture probe

(iii) HYPOTHETICAL:  No

(iv) ANTI-SENSE:  No

(vi) ORIGINAL SOURCE:  Synthetically prepared

(vii) IMMEDIATE SOURCE:  Same

(x)   PUBLICATION INFORMATION:  None

(xi) SEQUENCE DESCRIPTION:  SEQ ID NO:3:

ATCCTGGGAT TAAATAAAAT AGTAAGAATG TATAGCCCTA C    41